# FASCICULE DE BREVET EUROPEEN

(11) **EP 2 596 350 B1**
(45) Date de publication et mention de la délivrance du brevet: **14.05.2014**
(21) Numéro de dépôt: 11752585.7
(22) Date de dépôt: 18.07.2011
(51) Int. Cl.: G01N 33/49

(54) **APPAREIL ET PROCEDE DE DETECTION AUTOMATISEE DE PHASES POUR ANALYSE AUTOMATISEE**
VORRICHTUNG UND VERFAHREN ZUR AUTOMATISIERTEN ERKENNUNG VON PHASEN FÜR AUTOMATISIERTE ANALYSEN
APPARATUS AND METHOD FOR THE AUTOMATED DETECTION OF PHASES FOR AUTOMATED ANALYSIS

(30) Priorité: 22.07.2010 FR 1056003
(43) Date de publication de la demande: 29.05.2013
(73) Titulaire: Cybio France Sarl, 77176 Savigny-le-Temple (FR)
(72) Inventeur: FRAYSSINHES, Daniel, 30150 Roquemaure (FR); LOIRE, Yves, F-94510 La Queue en Brie (FR)
(74) Mandataire: Pontet Allano & Associes
(86) Numéro de dépôt international: PCT/FR2011/051709
(87) Numéro de publication internationale: WO 2012/010787

(56) Documents cités:
- WO-A1-94/14073
- WO-A2-00/08472
- US-A- 4 683 579
- US-A- 5 279 150
- US-A1- 2005 163 354
- US-A1- 2007 177 778

## Description

La présente invention concerne un appareil et un procédé de détection du niveau des différentes phases présentes dans au moins un tube ou récipient dont le contenu est destiné au chargement des différents puits d'un conteneur au format microplaque pour un système d'analyse automatisé. Selon l'invention, cette détection se fait par réflexion de lumière sur une zone ponctuelle de l'extérieur du tube, au cours d'un déplacement du tube le long d'un lecteur optique ou du lecteur optique le long du tube selon un mouvement rectiligne et de façon connue.

Elle concerne en outre un tel appareil ou procédé fonctionnant par éclairage en lumière monochrome et détection de la quantité de lumière réfléchie, avec reconnaissance des différences de phases par la variation brusque de la quantité de lumière réfléchie.

De nombreux procédés chimiques de laboratoire sont de plus en plus automatisés, par exemple des analyses chimiques ou de séquençage d'ADN. Les différents produits devant être mélangés entre eux sont manipulés par des matériels robotisés, et l'ensemble du processus est commandé et surveillé par informatique. L'essor des analyses ADN à grande échelle, par exemple par les procédés de type PCR, a accru les besoins en la matière et rend intéressant la moindre amélioration de productivité et de fiabilité des ces processus et des matériels correspondants.

Dans la mesure des possibilités techniques de chaque installation, on essaie en outre, autant que possible, de réaliser le processus en minimisant les opérations manuelles qui sont sources de perte de temps et de risques d'erreur.

Typiquement, on utilise une installation automatisée intégrée incluant un suivi informatisé des produits et échantillons traités. Une telle installation comprend un ou plusieurs actionneurs robotisés, par exemple un instrument de pipetage qui prélève une dose précise d'un liquide dans un récipient pour la transférer dans un autre récipient, où se fera ensuite une réaction. Ce récipient peut alors être déplacé dans un autre emplacement pour une autre opération, et/ou stocké dans un emplacement d'attente pendant la réaction, possiblement dans un réacteur assurant des conditions spécifiques de température, de pression, d'humidité, etc..

Pour des raisons de fiabilité d'analyse et de productivité, on utilise le plus souvent des récipients d'un type standardisé, appelé microplaque ou « microplate ». Un tel récipient comprend le plus souvent une surface monolithique percée d'un grand nombre de puits de quelques millimètres de diamètre qui sont indépendants les uns des autres. D'autres types de microplaques existent aussi qui sont ici englobés sous la même appellation, par exemple un panier ou rack comprenant des emplacements dans lesquels sont insérés autant de tubes individuels amovibles, et remplissant une fonction similaire aux puits d'une plaque monolithique. Selon les versions, une même plaque peut comporter par exemple 96 ou 384 puits. Plusieurs puits d'une même microplaque sont souvent traités en parallèle par des instruments de pipetage multi-têtes, qui peuvent être commandés ensemble ou séparément.

Ces plaques présentent toutes une même géométrie extérieure, en particulier dans leur empreinte inférieure. Cette géométrie est régie par un standard appelé « SBS » (ANSI/ SBS 1-2004), qui permet une compatibilité de toutes les plaques avec la plupart des robots et machines spécialisés dans ce domaine. Cette géométrie comprend par exemple une base rectangulaire qui présente deux angles chanfreinés coupés, et est munie d'un bourrelet dépassant légèrement à l'horizontale autour de l'embase. Cette forme standardisée permet à tous les robots compatibles d'utiliser un bras robotisé muni d'un emplacement compatible pour recevoir, saisir et maintenir toutes les plaques, de façon solide, précise et répétable.

Dans un certain nombre de cas, et en particulier pour la plupart des analyses de sang ou de liquides biologiques, l'analyse ou le traitement automatisé comprend une phase de séparation, par exemple par centrifugation ou décantation, qui permet de séparer les différents constituants existant au sein du liquide initialement prélevé.

Par exemple dans le cas du sang, le prélèvement initial est versé dans un tube à essais, aussi appelé éprouvette, qui est ensuite centrifugé. Le résultat de cette centrifugation donne une répartition des différents constituants sur plusieurs niveaux différents, formant les phases suivantes :
- en bas du tube se trouve une phase épaisse rouge sombre contenant les globules rouges ;
- au dessus se trouve une phase blanchâtre peu épaisse formant une sorte d'émulsion appelée « buffy coat », qui contient en grande partie des globules blancs ;
- en partie haute se trouve une phase liquide rouge plus claire formée du plasma, qui représente environ 55% du volume sanguin.

Pour utiliser un seul des constituants ainsi séparés, on utilise un instrument de pipetage que l'on fait descendre dans le tube jusqu'à la profondeur où se trouve le constituant visé, par exemple dans le buffy coat pour prélever des globules blancs. Le composant ainsi prélevé est alors versé dans un ou plusieurs récipients, par exemple pour une série de puits au sein d'une microplaque, opération qui est souvent appelée « chargement » de la microplaque.

Dans le tube de centrifugation, la position verticale des différentes phases varie selon de nombreux paramètres, tels que la quantité initiale de liquide ou le diamètre du tube. Pour pouvoir automatiser le prélèvement dans une phase déterminée, il est donc nécessaire au robot de disposer d'un appareil de détection des niveaux des différentes phases dans chacun des tubes à prélever.

Différents types d'appareil sont connus pour réaliser cette détection. Certains matériels mesurent par exemple la variation de la lumière transmise par le contenu du tube. Ces procédés présentent des inconvénients dus par exemple à la variabilité des facteurs de transmission. De plus, la transmission ne permet pas de distinguer entre des phases opaques même si elles contiennent des constituants différents. D'autres procédés mesurent une fluorescence émise par le contenu du tube sous excitation lumineuse, mais nécessitent pour cela des instruments relativement complexes et coûteux, et de forte puissance.

Le document US 4,683,579 propose de mesurer la dispersion d'une lumière de 400 à 1000 nanomètres incidente en lumière rasante, à un angle de l'ordre de 20°. Cette technique présente toutefois une précision pouvant être insuffisante, et représente autour du tube un encombrement non négligeable qui est gênant pour l'intégration dans un système robotisé.

Le document US 7,450,224 propose de réaliser une analyse graphique informatisée d'une image couleur complète du tube. Pour cela, le tube est saisi par une pince robotisée et amené dans une chambre d'imagerie contenant une caméra à capteur CCD couleur multipixels et un éclairage uniforme multidirectionnel. Le tube est extrait d'un rack positionné sur une table à déplacement XY.

Cette technique présente toutefois des inconvénients, par exemple de nécessiter des composants relativement coûteux et un traitement informatique complexe nécessitant une certaine puissance de calcul. De plus, un tel appareil occupe en permanence un certain espace et nécessite une table à déplacement robotisé pour pouvoir choisir le tube à prélever de façon automatisée.

Un but de l'invention est de pallier tout ou partie des inconvénients de l'art antérieur, et en en particulier sur les points suivants :
- simplicité, compacité, fiabilité ;
- souplesse d'utilisation et de programmation ;
- adaptation à la variabilité des tubes, de leur positionnement, à la présence de parties opaques, telles qu'une étiquette ;
- adaptation aux besoins, ou non, du processus réalisé ;
- possibilité d'utilisation dans un robot intégré.

De plus, il est intéressant de pouvoir minimiser l'occupation des actionneurs disponibles sur une installation robotisée, par exemple pour permettre une meilleure intégration de l'ensemble du processus d'analyse en étant moins limité par le nombre d'emplacements ou d'actionneurs disponibles.

### Exposé de l'invention

L'invention propose un appareil de détection de niveau des différentes phases présentes dans au moins un tube ou récipient, transparent au moins en partie et pour au moins certaines longueurs d'ondes, dont le contenu est destiné au chargement automatisé d'au moins un conteneur d'analyse pour un système d'analyse automatisé, typiquement pour le chargement des différents puits d'un conteneur au format microplaque. Selon l'invention, cet appareil comprend :
- des moyens pour déplacer ce tube le long d'un lecteur optique, ou ce lecteur optique le long de ce tube, de façon connue c'est-à-dire commandée ou mesurée ;
- des moyens d'enregistrement de données représentant la position verticale d'au moins un changement de phases au sein du contenu de ce tube.

De préférence, le dispositif est conçu avec une cinématique réalisant un déplacement du tube selon un mouvement rectiligne ou sensiblement, par exemple vertical.

Dans une variante, le mouvement peut être conçu pour réaliser un mouvement linéaire non rectiligne, et les moyens d'enregistrement sont agencés ou programmés pour adapter leur calcul à la trajectoire du tube, par exemple par comparaison avec une abaque ou par une formule de calcul tenant compte de la trajectoire du tube.

Selon l'invention, cet appareil fonctionne par mesure de la variation de longueur d'onde d'une lumière réfléchie sur le contenu de ce tube. Il est en outre muni d'une partie de fixation agencée pour lui permettre d'être lui-même maintenu et/ou manipulé en lieu et place d'un conteneur au format microplaque, par exemple par un bras robotisé prévu pour la manipulation de microplaques.

De préférence, l'appareil est doté de moyens de déplacement fonctionnant selon un seul axe de déplacement, voire d'un unique actionneur monoaxe de déplacement (en plus des moyens de préhension).

L'appareil peut ainsi être utilisé pour mesurer le contenu d'un récipient classique disposé parmi d'autres dans un rack standard, au sein d'un processus automatisé traitant et gérant la totalité de ces récipients.

Il est ainsi possible de programmer le prélèvement de toutes les différentes phases, dans chacun des récipients d'un rack, pour en charger une ou plusieurs microplaques et en réaliser l'analyse ou le traitement automatisé, en un seul processus global automatisé.

Le mode de lecture permet une conception relativement simple et compacte, ainsi qu'économique par rapport à d'autres techniques plus élaborées. Il permet en outre de détecter des différences entre plusieurs phases opaques dès lors qu'elles sont de couleurs différentes, contrairement aux techniques utilisant une transmission. Cette compacité rend plus facile la conception d'un appareil dont la géométrie est similaire au format microplaque et adapté aux manipulateurs correspondants.

Cette compatibilité mécanique avec le format microplaque permet d'intégrer facilement l'utilisation de cet appareil dans un cycle global de traitement automatisé. En effet, il est possible de le fixer sur un actionneur existant qui peut alors être programmé pour le déplacer et le positionner à volonté selon les besoins du processus.

L'appareil lui-même peut ainsi se passer d'actionneurs multiaxes et/ou à longue portée, puisqu'il peut être lui-même déplacé jusqu'à proximité du récipient à mesurer. Il est plus simple, compact et robuste.

En plaçant l'appareil dans un emplacement géré par le système robotisé, on limite ou supprime aussi les problèmes de manipulation et reprise de plaques ou de conteneurs dans le cycle automatisé, ainsi que l'espace occupé au sol. L'intégration devient plus souple et facile, en particulier lorsque l'on dispose d'une installation complète intégrée dotée d'une espace de travail restreint et compact.

De plus, une fois en place sur un emplacement microplaque ou « gripper » de l'installation robotisée, l'appareil dispose de ses propres moyens de préhension et déplacement du récipient à mesurer. On évite ainsi d'utiliser un deuxième emplacement ou actionneur du robot, ou d'en manquer si aucun autre n'est disponible.

De plus, chaque fois que cet appareil n'est pas nécessaire, il est alors possible de retirer l'appareil pour libérer un emplacement microplaque et économiser l'espace dans une installation robotisée.

Selon une particularité, l'appareil comprend une embase dont la périphérie inférieure, ou empreinte, présente une géométrie compatible avec le format microplaque. Ses moyens de déplacement sont en outre agencés pour accéder au tube à lire par au moins un déplacement du tube ou du lecteur optique situé dans une région à l'intérieur et en dessous de ladite empreinte.

De préférence, les moyens de déplacement présentent au moins une position dite rétractée dans laquelle ils ne dépassent pas en dessous ni à l'extérieur de l'empreinte au format microplaque. Ces moyens de déplacement sont par exemple escamotables dans une embase ne dépassant pas les contours extérieurs et inférieurs de l'empreinte au format microplaque, et de préférence à l'intérieur d'un carter supérieur de protection.

L'appareil prend ainsi peu de place lors des déplacements du bras qui le porte et facilite l'organisation du processus global d'analyse ou de traitement.

On voit qu'on obtient ainsi un appareil compact, robuste, souple d'utilisation et de programmation, facile à manier et à ranger sans dommages aussi bien par le robot qu'en dehors de son fonctionnement.

Dans un mode de réalisation préféré, l'appareil comprend des moyens de préhension du tube, par exemple une paire de pinces, lesquels présentent une géométrie déterminée pour leur permettre d'être insérés par le haut autour d'un tube disposé à l'intérieur d'une pluralité de tubes sensiblement parallèles entre eux au sein d'un rack de maintien, par exemple des tubes verticaux dans un rack horizontal.

Dans un autre mode de réalisation non représenté ici, les moyens de déplacement déplacent le lecteur optique le long d'un tube situé en dessous de cet appareil. Ces moyens de déplacement et ce lecteur optique présentent ensemble une géométrie déterminée pour leur permettre d'être déplacés depuis le haut, le long d'un tube disposé à l'intérieur d'une pluralité de tubes sensiblement parallèles entre eux (par exemple dans un même plan transversal, c'est à dire non longitudinal) au sein d'un conteneur de maintien (par exemple des tubes verticaux dans un rack horizontal).

Ce mode de réalisation peut aussi être combiné avec le précédent, par exemple dans une configuration où le tube et le lecteur se déplacent l'un par rapport à l'autre et tous les deux par rapport à l'embase, et/ou au bâti.

Selon l'invention, le lecteur optique comprend au moins un capteur détectant la longueur d'onde de la lumière réfléchie par le contenu du tube ou au moins par la surface extérieure de ce contenu, en une zone restreinte déterminée, mobile le long dudit tube lors du déplacement de lecture.

Selon une particularité de l'invention, le lecteur optique (14, 6) comprend une pluralité de modules de lecture optiques (6a à 6d) répartis dans plusieurs positions angulaires différentes autour du tube (9) et dans un même plan horizontal, agencés pour réaliser une mesure dans ces différentes positions angulaires.

Ces modules peuvent comprendre chacun un ou plusieurs capteurs, et/ou une source lumineuse.

Le lecteur optique peut aussi comprendre un ou plusieurs miroirs disposés autour du tube de façon à réfléchir la lumière issue de la source vers une pluralité de positions angulaires de mesure réparties autour du tube, et/ou de façon à renvoyer la lumière réfléchie par le contenu du tube vers un même capteur depuis une pluralité de positions angulaires autour du tube.

En combinant ainsi plusieurs points de mesure autour du tube, l'invention permet en particulier de trouver au moins une position de lecture exploitable même si le tube n'est pas transparent sur toute sa périphérie, par exemple en raison d'une étiquette collée sur sa paroi.

Selon une particularité préférée de cet appareil :
- d'une part il comprend une ou plusieurs source lumineuses n'émettant que dans une partie déterminée du spectre lumineux ; et
- d'autre part le lecteur optique comprend un ou plusieurs modules de lecture comprenant chacun un unique capteur monopixel sensible à la couleur de ladite source lumineuse, par exemple une simple photodiode unique.

Les moyens d'enregistrement sont alors agencés pour utiliser la quantité de lumière réfléchie reçue par ce ou ces capteurs pour reconnaître le changement de longueur d'onde de cette lumière réfléchie.

A chaque instant la lumière colorée est réfléchie plus ou moins selon la couleur de la phase éclairée. La ou les longueurs d'onde de la lumière colorée sont choisies en fonction des différences de couleur entre les différentes phases dont on veut détecter la séparation.

Ainsi, si une phase est rouge et l'autre blanche, une lumière bleue sera réfléchie beaucoup plus par la phase blanche que par la phase rouge, qui apparaîtra ainsi beaucoup plus sombre.

En détectant les variations marquées de la quantité de lumière réfléchie, toutes longueurs d'onde confondues, on peut donc repérer le changement de couleur de la zone dont provient la lumière réfléchie mesurée.

On comprend que ce type de détection permet une conception simple, compacte et économique, en particulier du point de vue de l'électronique, ce qui concourt à la compacité de l'ensemble.

Selon un autre aspect, l'invention propose aussi un procédé de détection de niveau des différentes phases présentes dans au moins un tube ou récipient destiné au chargement des différents puits d'au moins un conteneur pour un système d'analyse automatisé, ledit procédé comprenant :
- un déplacement, selon un mouvement rectiligne et de façon connue, de ce tube le long d'un lecteur optique, ou de ce lecteur optique le long de ce tube ;
- un enregistrement de données représentant la position verticale d'au moins un changement de phases au sein du contenu de ce tube.

Selon l'invention, cet enregistrement comprend un enregistrement de la quantité de lumière issue d'une source colorée et réfléchie par le contenu dudit tube en au moins un point déterminé mobile le long dudit tube, c'est-à-dire mobile relativement au tube.

La mention d'un point de lecture doit s'entendre comme signifiant une zone restreinte, par exemple du point de vue des dimensions du tubes ou de la hauteur des phases mesurées dans le tube. La mesure peut aussi se faire en plusieurs points déterminés, c'est-à-dire alors en plusieurs zones restreintes isolées les unes des autres.

Des modes de réalisation variés de l'invention sont prévus, intégrant selon l'ensemble de leurs combinaisons possibles les différentes caractéristiques optionnelles exposées ici.

D'autres particularités et avantages de l'invention ressortiront de la description détaillée d'un mode de mise en oeuvre nullement limitatif, et des dessins annexés sur lesquels :
- les FIGURE 1, FIGURE 2, FIGURE 3 et FIGURE 4 sont des vues en perspectives illustrant un exemple de mode de réalisation de l'invention, dans les positions suivantes :
   ○ FIGURE 1 en position basse lors de la saisie d'un tube,
   ○ FIGURE 2 lors de la lecture en cours de remontée,
   ○ FIGURE 3 en position haute en fin de lecture, et
   ○ FIGURE 4 en position rétractée en dehors d'une opération de lecture ;
- la FIGURE 5 est un graphique représentant les résultats de la mesure d'intensité lumineuse réfléchie, qui sont interprétés par les moyens d'enregistrement pour identifier la position des changements de phase au sein du tube ;
- la FIGURE 6 est une vue schématique de dessus illustrant un exemple de lecteur optique à une source et quatre modules de lecture munis chacun d'un capteur monopixel, mesurant en quatre points différents ;
- la FIGURE 7 est un schéma fonctionnel illustrant des opérations de chargement dans un procédé d'analyse ou de traitement, comprenant une détection de niveaux de phases dans un mode de réalisation de l'invention ;
- les FIGURE 8, FIGURE 9 et FIGURE 10 sont des vues partielles en perspective d'une installation automatisée dans différentes phases d'opérations de chargement selon la FIGURE 7, avec :
   ○ en FIGURE 8 : détection des niveaux de phases dans l'un des tubes d'un rack de tubes,
   ○ en FIGURE 9 : prélèvement d'une phase dans ce tube en fonction des résultats de la détection, et
   ○ FIGURE 10 : chargement d'une microplaque avec la phase prélevée.

Dans le mode de réalisation ici décrit, l'appareil forme un « module de détection » qui peut être facilement monté sur un emplacement microplaque pour mesurer avec précision les hauteurs des différentes phases d'un tube de prélèvement sanguin centrifugé. Pour effectuer cette mesure le « bloc optique » parcourra le tube dans toute sa hauteur. Durant cette translation, la réflexion de la lumière est mesurée et la position du bloc optique relativement au tube au moment des changements de niveau de réflexion déterminera les hauteurs des différentes phases.

Le module de détection dispose d'un dispositif de préhension des tubes de prélèvement sanguin, permettant de saisir les tubes jusqu'à une distance par exemple de 100 mm en dessous du module, pour les faire traverser intégralement le module de détection pour effectuer la détection de phase. Ce dispositif de préhension se composera par exemple d'un « levier » et d'un « gripper ».

Une fois refermé le module a le format complet d'une microplaque (Format SBS)

Le détecteur de phases peut être utilisé avec un bras robotique au sein d'une plateforme robotique, Il peut aussi être utilisé seul, en ce cas c'est l'opérateur qui alimentera le détecteur à la main, lequel sera monté sur une embase isolée permettant au tube de monter et descendre le long de l'axe perpendiculaire au faisceau lumineux.

La détection du module de détection de phase est basée sur un principe d'absorption/réflexion d'une longueur d'onde déterminée par les différentes phases d'un tube de prélèvement sanguin centrifugé. Les différentes phases étant, à partir du fond du tube : rouge sombre (globules rouges), blanchâtre (composés cellulaires dont environ 45% de globules blancs), et rougeâtre (plasma) ; la longueur d'onde choisie ici se trouve dans la région bleue du spectre visible (400 à 500nm). Cette longueur d'onde est ainsi absorbée par les phases globules rouges et plasma et réfléchie par la phase de composés cellulaires.

Le tube est entraîné selon un mouvement perpendiculaire à l'axe du faisceau lumineux à l'aide d'un bras motorisé, par exemple commandé par un microprocesseur. Cela permet de connaître la position exacte de chaque phase par rapport au sommet et/ou au fond du tube

La mesure se fait par détection de la quantité de lumière réfléchie au cours du déplacement

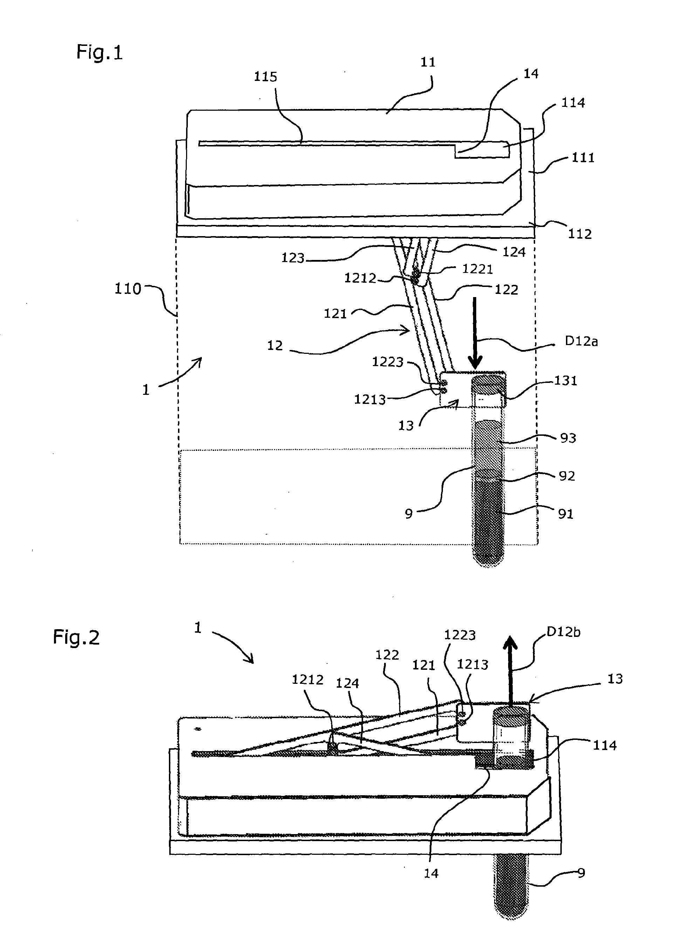

Les FIGURE 1, FIGURE 2, FIGURE 3 et FIGURE 4 illustrent un exemple de mode de réalisation de l'invention, tel qu'actuellement préféré.

Dans ce mode de réalisation, l'appareil comprend une embase 11 dont la périphérie inférieure, ou empreinte, présente une géométrie compatible avec le format microplaque. Cette géométrie inclut une forme rectangulaire avec des dimensions normalisées pour cette embase, et comprend :
- d'une part un bourrelet rectangulaire 111 dépassant autour de l'embase, et
- d'autre part deux angles adjacents de l'embase 11 présentant chacun un pan coupé vertical 112 de dimensions normalisées, encadrant un petit côté de cette embase.

Dans cet exemple de mode de réalisation, les moyens de déplacement 12, 13 comprennent des moyens de préhension 13 du tube 9, ici une pince formée par une platine 13 portant un actionneur muni d'une bride 131 venant serrer le tube contre cette platine.

Ces moyens de préhension sont déplacés par un mécanisme 12 à tringles mobiles 121, 122, 123, 124 mobiles à l'intérieur d'une fente rectiligne traversante 115 ménagée dans l'embase 11.

Ces tringles sont reliées entre elles par des articulations pivots 1213, 1223, 1212, 1221 d'axes perpendiculaires au plan de déplacement, réalisant un pantographe de déplacement des moyens de préhension 13 ou du lecteur optique selon une direction rectiligne D12a et D12b incluse dans ce plan de déplacement P12 et parallèle à l'axe du tube 9 à lire.

Ce mécanisme 12 comprend en particulier deux tringles principales 121, 122 articulées en parallélogramme entre l'embase et la platine de préhension 13. Ce mécanisme 12 comprend en outre deux tringles secondaires 123, 124 plus courtes, elles-mêmes articulées en parallélogramme entre l'embase 11 et une partie intermédiaire 1221, 1212 voire centrale des tringles principales 121, 122.

Du côté de l'embase, les extrémités des deux parallélogrammes sont déplacées l'une par rapport à l'autre par des moyens d'actionnement pour faire varier la distance entre elles, déplaçant ainsi en hauteur les moyens de préhension 13. Cette variation est faite par exemple par une vis sans fin actionnée par un moteur et faisant coulisser l'extrémité du parallélogramme principal 121, 122 le long de la fente 115 de l'embase, pendant que l'extrémité du parallélogramme secondaire pivote en un point fixe à l'intérieur de cette fente.

Sur cette figure, on voit que les moyens de déplacement 12, 13 permettent d'accéder au tube 9 à lire par :
- un déplacement D12a vers le bas pour approcher et saisir le tube, puis
- un déplacement D12b vers le haut avec le tube 9 qui permet de l'extraire de son emplacement longitudinalement à son axe, puis
- un autre déplacement vers le bas (non représenté) pour remettre le tube en place.

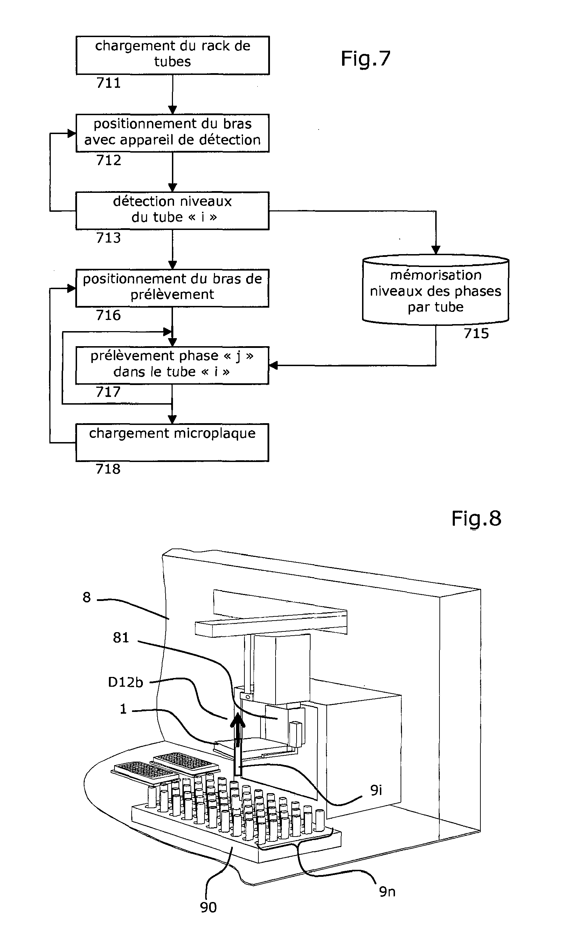

Ces différents déplacements sont tous situés dans une région 110, figurée en pointillés, située à l'intérieur de l'empreinte formée par la géométrie au format microplaque 111, 112 de l'embase 11. La saisie et l'extraction du tube se font plus particulièrement en dessous de cette embase, ce qui permet d'accéder à un ou plusieurs tubes depuis le haut, même lorsqu'ils sont rangés de façon serrée dans un rack de maintien, comme illustré en FIGURE 8.

A l'intérieur de l'empreinte (111) au format microplaque, la fente 115 du mécanisme de déplacement 12 se prolonge par une ouverture traversante (114) suffisamment large pour laisser passer les moyens de préhension et le tube 9 au cours d'un déplacement vertical complet.

Des moyens de lecture 14 sont disposés dans les parois de cette ouverture traversante 114, de façon à effectuer la mesure lors du passage D12b du tube au travers de cette ouverture, dans un sens ou dans l'autre.

Les moyens de lecture 14 sont ainsi bien protégés, peu vulnérables et peu encombrants.

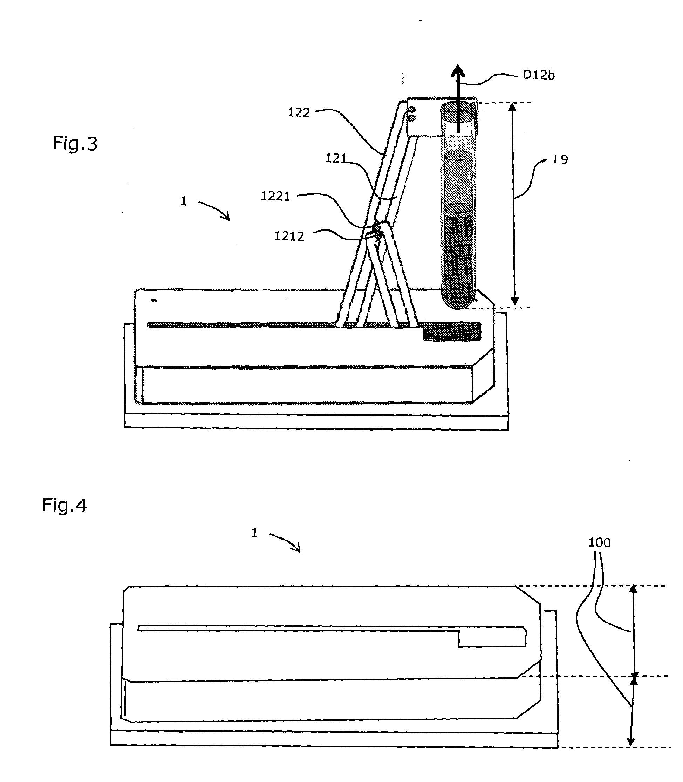

Ainsi qu'on le voit en FIGURE 4, les moyens de déplacement 12, 13 sont escamotables dans une position dite rétractée dans laquelle ils ne dépassent pas en dessous ni à l'extérieur de l'empreinte au format microplaque 111, non plus qu'au dessus d'un carter de protection sur le dessus de l'embase, l'ensemble ayant par exemple les dimensions normalisées 100 d'une microplaque.

Lorsqu'il n'est pas utilisé, l'appareil forme approximativement un parallélépipède rectangle simple et compact, sans parties dépassantes en dehors du bourrelet inférieur 111, facile à manipuler et à ranger sans dommages.

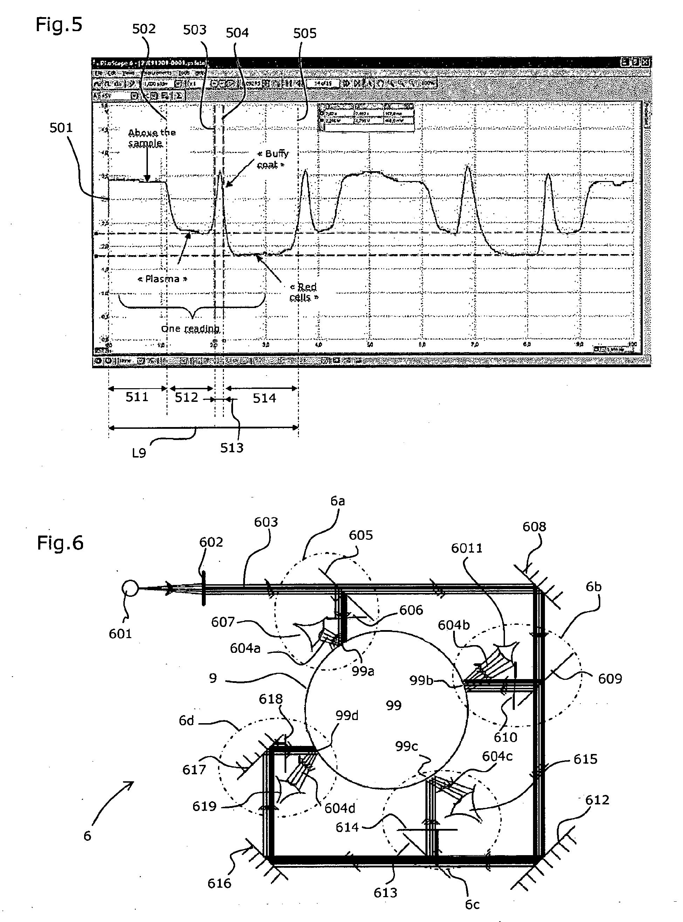

La FIGURE 6 illustre un exemple de lecteur optique à une source et quatre modules de lecture munis chacun d'un capteur monopixel, mesurant en quatre points différents.

Dans cet exemple, l'appareil comprend d'une part une source lumineuse 601 n'émettant que dans une partie déterminée du spectre lumineux, et d'autre part le lecteur optique 14, 6. Ce lecteur optique comprend ici quatre modules de lecture 6a à 6d comprenant chacun un unique capteur monopixel 607, 611, 615, 619 sensible à la couleur de cette source lumineuse, par exemple une photodiode.

Les différents capteurs du lecteur optique 14, 6 détectent la longueur d'onde de la lumière réfléchie 604a à 604d par le contenu 99 (essentiellement par sa surface extérieure) du tube 9 en une zone restreinte 99a à 99d déterminée formant chacune un »point » de lecture, chacun mobile le long de ce tube lors du déplacement de lecture D12b.

Dans cet exemple, la source lumineuse 601 éclaire le point de lecture le plus éloigné 99d par un jeu de miroirs de renvoi 608, 612, 616 et 618 formant un chemin optique d'acheminement de la lumière d'éclairage 603 après collimation par une lentille 602.

Sur ce chemin de lumière d'éclairage 603, trois miroirs semi réfléchissants 605, 609 et 613 dévient chacun une partie de la lumière d'éclairage 603 pour éclairer chacun un des trois autres points de mesure 99a, 99b et 99c.

Chacun de ces points de lecture reçoit la lumière d'éclairage à travers des moyens de restriction qui permettent de limiter la surface éclairée à la surface du contenu 99 du tube, par exemple des moyens de collimation ou un diaphragme 606, 610, 614 et respectivement 618. Il serait aussi possible d'utiliser une source suffisamment étroite comme une diode laser. La zone restreinte est par exemple de dimensions inférieures à un cercle de 0,5 voire 0,2mm de diamètre.

On éclaire ainsi les différentes couches à l'aide d'un faisceau lumineux qui réfléchiront la lumière de manière différentes selon leur constitution.

Chaque module de lecture 6a à 6d comprend un unique capteur monopixel 607, 611, 615 et respectivement 619, qui mesure la lumière réfléchie 604a à 60d par son point de lecture respectif 99a à 99d.

Les moyens d'enregistrement (non représentés ici) sont agencés et programmés pour utiliser la quantité de lumière réfléchie 604a à 604d reçue par ces capteurs pour reconnaître le changement de longueur d'onde de cette lumière réfléchie.

Dans le cas de plusieurs capteurs, une sélection peut être faite entre les différentes lectures, ou une opération mathématique ou logique pour fournir un résultat unique.

On obtient ainsi des « photocapteurs multiplexés » permettant la mesure de la lumière réfléchie couvrant une circonférence de 60% minimum de ce tube.

Les différentes couches cellulaires absorbent une quantité de lumière différente selon leur constitution et réfléchissent ainsi une quantité de lumière inversement proportionnelle à la quantité de lumière absorbée.

La FIGURE 5 représente les résultats de la mesure d'intensité lumineuse réfléchie, qui sont interprétés par les moyens d'enregistrement pour identifier la position des changements de phase au sein du tube.

Ainsi qu'il est visible sur la figure, la valeur de la quantité de lumière réfléchie varie au cours du déplacement du tube par rapport au lecteur.

A partir de la gauche dans cette figure, on voit un premier front montant 501 correspondant à la détection du haut du tube, suivi d'un palier 511 correspondant à la partie vide en haut du tube.
- Un premier front descendant 502 suivi d'un palier 512 correspond à la détection de la phase 93 rouge clair du plasma.
- Deux fronts successifs inversés 503 et 504 forment ensuite une région 513 suffisamment étroite pour prendre la forme d'un pic, correspondant à la hauteur de la phase blanchâtre d'émulsion ou buffy coat 92.
- Le front descendant 504 et le palier 514 indiquent ensuite la phase 91 rouge sombre des globules rouges.
- Le front montant 505 suivant indique le passage de l'extrémité basse du tube 9 devant le lecteur optique 14.

Cette analyse des fronts et paliers est programmée pour fournir une mesure des hauteurs et niveaux des différentes phases présentes dans le contenu du tube 9, ainsi que la hauteur totale L9 du tube.

En déplaçant le tube sur toute sa longueur, on peut ainsi connaître la hauteur totale du tube et s'affranchir de sa variabilité d'un tube à l'autre. Le dispositif est alors compatible avec n'importe quelle hauteur de tube dans la mesure où la hauteur est suffisante pour être attrapé par le « gripper » du détecteur.

Un calculateur génère des données numériques correspondant aux résultats de la mesure, par exemple un fichier indiquant :
- la hauteur du tube (différence entre le point le plus haut du tube et le fond du tube) ;
- la quantité de lumière réfléchie par rapport à une hauteur en mm définie préalablement.

Ce fichier est généré par exemple dans un format « txt », « csv », « xml », ou tout format compatible et pouvant être retraité par un pipeteur automatique.

Il est ainsi possible de pipeter de manière discriminante l'une ou l'autre phase, voire toutes les trois, de façon précise et reproductible de tube à tube, et indépendamment pour chacun des tubes en fonction des besoins du traitement.

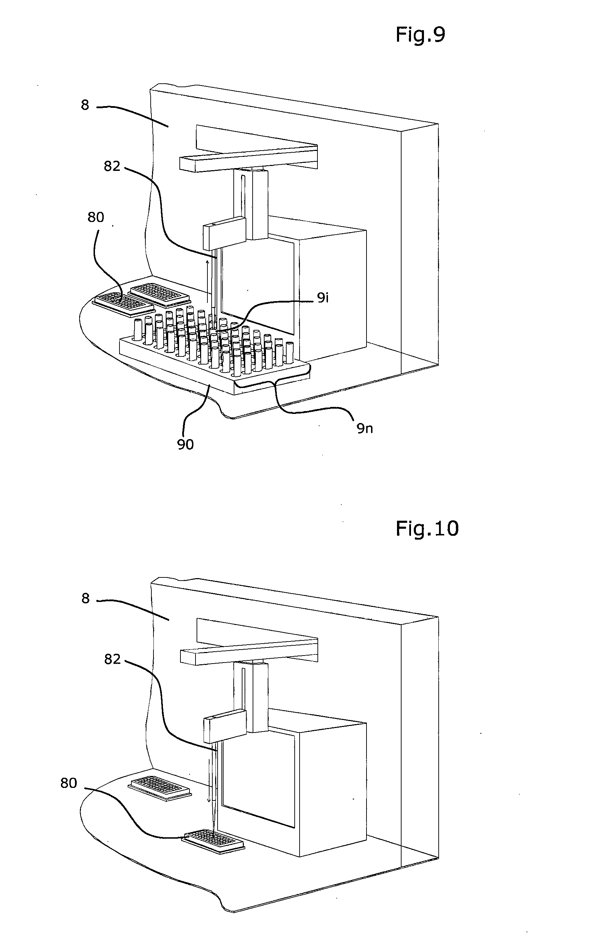

En FIGURE 7 sont illustrées des opérations de chargement dans un procédé d'analyse ou de traitement dans une installation automatisée, comprenant une détection de niveaux de phases dans un mode de réalisation de l'invention. Les FIGURE 8, FIGURE 9 et FIGURE 10 illustrent certaines opérations réalisées au cours de ce procédé.

Une étape de chargement 711 comprend le chargement d'un rack 90 comprenant un jeu de tubes 9n, dans l'espace de travail d'une installation automatisée 8. Ce rack 90 est placé dans la d'accès d'un appareil de détection 1 selon l'invention, lequel est fixé au gripper d'un bras robotisé 81 de cette installation 8.

Le bras robotisé 81 positionne 712 le détecteur 1 au dessus d'un tube 9i choisi par le programme.

Le détecteur 1 extrait D12b le tube choisi 9i du rack et effectue 713 la mesure des niveaux des différentes phases contenues dans ce tube 9i, ainsi que la hauteur du tube.

Ces informations sont mémorisées 715 associées aux références de ce tube 9i.

Ces étapes de détection et mémorisation peuvent être répétées automatiquement pour la totalité des tubes 9n contenus dans le rack 90.

Une fois que tous les tubes ont été mesurés, un appareil automatisé de pipetage est positionné au dessus du rack 90, ou inversement.

Pour chaque tube 9i choisi, une pointe de pipetage 82 est positionnée 716 au dessus du tube, et plongée dans ce tube à la profondeur nécessaire pour accéder à la phase « j » choisie, en se basant sur les informations de niveaux des phases et de hauteur précédemment mémorisées 715 pour ce même tube 9i déterminé. Une dose choisie de la phase choisie est alors prélevée 717 par la pointe de pipetage.

Cette pointe de pipetage 82 va alors charger un conteneur d'analyse avec la dose prélevée de la phase « j » choisie du tube 9i choisi, par exemple dans un ou plusieurs puits d'une microplaque 80.

Ces étapes de prélèvement et chargement peuvent être répétées automatiquement pour la totalité des tubes 9n contenus dans le rack 90.

Le procédé de chargement comprend ainsi une étape robotisée de prélèvement 717 dans le même tube 9i détecté, commandée ou contrôlée à partir des informations 715 mémorisées lors de l'étape de détection 713.

On comprend que l'invention permet une sélection et un prélèvement automatisé d'une ou plusieurs phases dans un ou plusieurs tubes d'échantillons, avec une bonne adaptation des procédés automatisés à la variabilité des tubes, de leur positionnement, à la présence de parties opaques, telles qu'une étiquette.

Bien sûr, l'invention n'est pas limitée aux exemples qui viennent d'être décrits et de nombreux aménagements peuvent être apportés à ces exemples sans sortir du cadre de l'invention.

## Revendications

1. Appareil (1) de détection de niveau des différentes phases (91, 92, 93) présentes dans au moins un tube (9) ou récipient transparent au moins en partie, destiné au chargement automatisé d'au moins un conteneur d'analyse (80) pour un système d'analyse automatisé (8), **caractérisé en ce qu'**il comprend :
- des moyens (12, 13) pour déplacer, selon un mouvement (D12) connu, ledit tube (9) le long d'un lecteur optique (14, 6), ou ledit lecteur optique le long dudit tube ;
- des moyens d'enregistrement de données représentant la position verticale d'au moins un changement de phases au sein du contenu dudit tube ;
ledit appareil fonctionnant par mesure de la variation de longueur d'onde d'une lumière réfléchie (64a à 64d) sur le contenu (99) dudit tube (9), et étant muni d'une partie de fixation (112) agencée pour lui permettre d'être lui-même maintenu et/ou manipulé en lieu et place d'un conteneur (8) au format microplaque.

2. Appareil selon la revendication précédente, **caractérisé en ce qu'**il comprend une embase (11) dont la périphérie inférieure, ou empreinte, présente une géométrie (111, 112) compatible avec le format microplaque ; et **en ce que** les moyens de déplacement (12, 13) sont agencés pour accéder au tube (9) à lire par au moins un déplacement (D12b) rectiligne du tube (9) ou du lecteur optique situé dans une région (110) à l'intérieur et en dessous de ladite empreinte.

3. Appareil selon l'une quelconque des revendications précédentes, **caractérisé en ce que** les moyens de déplacement (12, 13) présentent au moins une position dite rétractée dans laquelle ils ne dépassent pas en dessous ni à l'extérieur de l'empreinte au format microplaque (111).

4. Appareil selon l'une quelconque des revendications précédentes, **caractérisé en ce que** les moyens de déplacement (12, 13) comprennent des moyens de préhension (13) du tube (9) déplacés par un mécanisme (12) à tringles mobiles (121, 122, 123, 124), dans un même plan de déplacement (P12) ayant une composante verticale et autour d'articulations pivots (1213, 1223, 1212, 1221) d'axes perpendiculaires audit plan de déplacement, réalisant un pantographe de déplacement des moyens de préhension (13) ou du lecteur optique selon une direction rectiligne (D12a, D12b) incluse dans ledit plan de déplacement et parallèle à l'axe du tube (9) à lire.

5. Appareil selon l'une quelconque des revendications précédentes, **caractérisé en ce qu'**il comprend des moyens de préhension (13) du tube (9), lesquels présentent une géométrie déterminée pour leur permettre d'être insérés par le haut (D12a) autour d'un tube disposé à l'intérieur d'une pluralité de tubes (9 à 9n) sensiblement parallèles entre eux au sein d'un rack de maintien (90).

6. Appareil selon l'une quelconque des revendications précédentes, **caractérisé en ce qu'**il comprend à l'intérieur de l'empreinte (111) au format microplaque au moins une ouverture traversante (114), et **en ce que** les moyens de déplacement (12, 13) sont agencés pour déplacer les moyens de préhension (13) avec le tube (9) au travers de ladite ouverture (pendant ou avant ou après) au moins en partie au cours d'un déplacement de lecture (D12b).

7. Appareil selon l'une quelconque des revendications précédentes, caractérisé en ce les moyens de déplacement déplacent le lecteur optique le long d'un tube situé en dessous dudit appareil, lesdits moyens de déplacement et ledit lecteur optique présentant ensemble une géométrie déterminée pour leur permettre d'être déplacés depuis le haut le long d'un tube disposé à l'intérieur d'une pluralité de tubes sensiblement parallèles entre eux au sein d'un conteneur de maintien.

8. Appareil selon l'une quelconque des revendications précédentes, **caractérisé en ce que** le lecteur optique (14, 6) comprend au moins un capteur (607, 611, 614, 619) détectant la longueur d'onde de la lumière réfléchie (604a à 604d) par le contenu (99) du tube (9) en une zone restreinte (99a à 99d) déterminée, mobile le long dudit tube lors du déplacement de lecture (D12b).

9. Appareil selon l'une quelconque des revendications précédentes, **caractérisé en ce qu'**il comprend au moins une source de lumière agencée pour éclairer une zone restreinte du contenu du tube, ou des moyens de restriction (606, 6010, 614, 618) de l'éclairage et/ou de la lecture à une zone restreinte (99a à 99d) du contenu (99) du tube (9).

10. Appareil selon l'une quelconque des revendications précédentes, **caractérisé en ce que**
d'une part il comprend d'une part une ou plusieurs sources lumineuses (601) n'émettant que dans une partie déterminée du spectre lumineux, et
d'autre part le lecteur optique (14, 6) comprend un ou plusieurs modules de lecture (6a à 6d) comprenant chacun un unique capteur monopixel (607, 611, 615, 619) sensible à la couleur de ladite source lumineuse,
les moyens d'enregistrement étant agencés pour utiliser la quantité de lumière réfléchie (604a à 604d) reçue par le ou lesdits capteurs pour reconnaître le changement de longueur d'onde de ladite lumière réfléchie.

11. Appareil selon l'une quelconque des revendications précédentes, **caractérisé en ce que** le lecteur optique (14, 6) comprend une pluralité de modules de lecture optiques (6a à 6d) répartis dans plusieurs positions angulaires différentes autour du tube (9) et dans un même plan horizontal agencés pour réaliser une mesure dans ces différentes positions angulaires.

12. Appareil selon l'une quelconque des revendications précédentes, **caractérisé en ce que** le lecteur optique (14, 6) comprend un ou plusieurs miroirs (605, 608, 609, 612, 613, 616, 617) disposés autour du tube (9) de façon à réfléchir la lumière (603) issue de la source (601) vers une pluralité de positions angulaires de mesure (99a à 99d) réparties autour du tube, et/ou de façon à renvoyer la lumière réfléchie par le contenu du tube vers un même capteur depuis une pluralité de positions angulaires autour du tube.

13. Procédé de détection de niveau des différentes phases (91, 92, 93) présentes dans au moins un tube (9) ou récipient destiné au chargement des différents puits d'au moins un conteneur (80) pour un système d'analyse automatisé (8), ledit procédé comprenant :
- un déplacement (D12b), selon un mouvement rectiligne et de façon connue, dudit tube (9) le long d'un lecteur optique (16, 4), ou dudit lecteur optique le long dudit tube ;
- un enregistrement de données représentant la position verticale d'au moins un changement de phases au sein du contenu dudit tube ;
ledit enregistrement comprenant un enregistrement de la quantité de lumière issue d'une source colorée (601) et réfléchie (604a à 604d) par le contenu (99) dudit tube en au moins un point déterminé (99a à 99d), mobile le long dudit tube,
ledit procédé étant **caractérisé en ce qu'**il est mis en oeuvre par un appareil de détection (1) qui :
d'une part est muni d'une partie de fixation (111, 112) agencée pour lui permettre d'être lui-même maintenu et/ou manipulé en lieu et place d'un conteneur au format microplaque ; et
d'autre part comprend des moyens (12, 13) pour déplacer, selon un mouvement rectiligne (D12b) et de façon connue, ledit tube (9) le long d'un lecteur optique (14, 6), ou ledit lecteur optique le long dudit tube.

14. Procédé selon la revendication précédente, ledit procédé comprenant en outre :
- une étape de positionnement (712) d'un membre robotisé (81) comportant un emplacement au format microplaque accueillant ledit appareil de détection (1), de façon à pouvoir effectuer une détection sur un tube déterminé choisi (9) au sein d'une pluralité de tubes (9n) sensiblement parallèles entre eux disposés au sein d'un rack de maintien (90);
- une étape de détection (713) des niveaux du tube choisi, comprenant :
- soit une opération d'extraction vers le haut hors dudit conteneur et un déplacement du tube choisi par rapport au lecteur optique,
- soit une opération de déplacement du lecteur optique par insertion vers le bas le long du tube choisi,
- soit une combinaison de ces opérations.

15. Procédé selon l'une quelconque des revendications 13 à 14, **caractérisé en ce qu'**il comprend en outre une analyse de la quantité de lumière réfléchie (604a à 604d), paramétrée pour détecter la position d'au moins une extrémité du tube (9), et/ou pour calculer la longueur dudit tube.

## Patentansprüche

1. Gerät (1) zur Erkennung des Stands der verschiedenen, in mindestens einem wenigstens teilweise durchsichtigen Rohr (9) oder Behälter vorliegenden Phasen (91, 92, 93), für das automatisierte Bestücken mindestens eines Analysebehälters (80) für eine automatisierte Analysevorrichtung (8) bestimmt, **dadurch gekennzeichnet, dass** es umfasst:
- Einrichtungen (12, 13) zum Verlagern, gemäß einer bekannten Bewegung (D12), des genannten Rohrs (9) entlang eines optischen Lesegeräts (14, 6) oder des genannten optischen Lesegeräts entlang des genannten Rohrs;
- Aufzeichnungsmittel für Daten, die die vertikale Position mindestens einer Phasenänderung innerhalb des Inhalts des genannten Rohrs darstellen;
wobei das genannte Gerät durch Messung der Änderung in der Wellenlänge eines reflektierten Lichts (64a - 64d) auf dem Inhalt (99) des genannten Rohrs (9) funktioniert und mit einem dergestalt ausgebildeten Befestigungsteil (112) ausgestattet ist, dass es selber anstelle eines Behälters (8) im Mikroplatte-Format gehalten und/oder gehandhabt werden kann.

2. Gerät nach dem vorhergehenden Anspruch, **dadurch gekennzeichnet, dass** es einen Sockel (11) enthält, dessen unterer Rand, der sogenannte Abdruck, eine mit dem Mikroplatte-Format kompatible Geometrie (111, 112) aufweist, und dadurch, dass die Verlagerungseinrichtungen (12, 13) derart angeordnet sind, um zum zu lesenden Rohr (9) über mindestens eine geradlinige Verlagerung (D12b) des Rohrs (9) oder des optischen Lesegeräts zu gelangen, das sich in einem Bereich (110) innerhalb und unterhalb des genannten Abdrucks befindet.

3. Gerät nach einem der vorhergehenden Ansprüche, **dadurch gekennzeichnet, dass** die Verlagerungseinrichtungen (12, 13) mindestens eine sogenannte zurückgezogene Position aufweisen, in welcher sie weder unterhalb noch außerhalb des Abdrucks mit Mikroplatte-Format (111) herausragen.

4. Gerät nach einem der vorhergehenden Ansprüche, **dadurch gekennzeichnet, dass** die Verlagerungseinrichtungen (12, 13) Greifeinrichtungen (13) für das Rohr (9) umfassen, welche durch einen Mechanismus (12) mit beweglichem Gestänge (121, 122, 123, 124) in einem und demselben Bewegungsplan (P12) mit einer vertikalen Koordinate und um Drehgelenke (1213, 1223, 1212, 1221) von Achsen senkrecht zum besagten Verlagerungsplan herum verlagert werden, einen Verlagerungspantograph der Greifeinrichtungen (13) oder des optischen Lesegeräts gemäß einer gradlinigen Richtung (D12a, D12b), in dem genannten Verlagerungsplan eingeschlossen und parallel zur Achse des zu lesenden Rohrs (9), ausführend.

5. Gerät nach einem der vorhergehenden Ansprüche, **dadurch gekennzeichnet, dass** es Greifeinrichtungen (13) für das Rohr (9) umfasst, die eine bestimmte Geometrie aufweisen, sodass sie von oben (D12a) um ein Rohr eingeführt werden können, das innerhalb einer Vielzahl von im Wesentlichen parallel zu einander innerhalb eines Haltegestells (90) gelagerten Rohren (9 - 9n) angeordnet ist.

6. Gerät nach einem der vorhergehenden Ansprüche, **dadurch gekennzeichnet, dass** es innerhalb des Abdrucks (111) mit Mikroplatte-Format mindestens eine durchgehende Öffnung (114) enthält, und dass die Verlagerungseinrichtungen (12, 13) derart ausgebildet sind, um die Greifeinrichtungen (13) samt dem Rohr (9) durch die genannte Öffnung hindurch (während oder vorher oder danach) mindestens teilweise im Laufe einer Verlagerung des Lesevorgangs (D12b) zu bewegen.

7. Gerät nach einem der vorhergehenden Ansprüche, **dadurch gekennzeichnet, dass** die Verlagerungseinrichtungen das optische Lesegerät entlang eines unterhalb des genannten Geräts gelagerten Rohrs verlagern, wobei die genannten Verlagerungseinrichtungen und das genannte optische Lesegerät zusammen eine derart bestimmte Geometrie aufweisen, um von oben entlang eines Rohrs bewegt werden zu können, das innerhalb einer Vielzahl von in einem Haltebehälter im Wesentlichen parallel zu einander gelagerten Rohren angeordnet ist.

8. Gerät nach einem der vorhergehenden Ansprüche, **dadurch gekennzeichnet, dass** das optische Lesegerät (14, 6) mindestens einen Sensor (607, 611, 614, 619) umfasst, der die Wellenlänge des durch den Inhalt (99) des Rohrs (9) reflektierten Lichts (604a - 604d) in einem eingeschränkten, bestimmten Bereich (99a - 99d) erfasst und entlang des genannten Rohrs während der Verlagerung des Lesevorgangs (D12b) beweglich ist.

9. Gerät nach einem der vorhergehenden Ansprüche, **dadurch gekennzeichnet, dass** es wenigstens eine derart angeordnete Lichtquelle enthält, um einen eingeschränkten Bereich des Rohrinhalts zu beleuchten, oder Einrichtungen zur Einschränkung (606, 6010, 614, 618) der Beleuchtung und/oder des Lesevorgangs auf einen eingeschränkten Bereich (99a - 99d) des Inhalts (99) des Rohrs (9).

10. Gerät nach einem der vorhergehenden Ansprüche, **dadurch gekennzeichnet, dass**
es zum einen eine oder mehrere Lichtquellen (601) umfasst, die nur in einem bestimmten Teil des Lichtspektrums ausstrahlen, und
zum anderen das optische Lesegerät (14, 6) eins oder mehrere Lesemodule (6a - 6d) umfasst, jeweils einen einzigen Monopixelsensor (607, 611, 615, 619) enthaltend, der auf die Farbe der genannten Lichtquelle empfindlich ist,
wobei die Aufzeichnungseinrichtungen derart ausgebildet sind, um die Menge des reflektierten Lichts (604a - 604d) zu verwenden, die von dem oder den genannten Sensoren empfangen wird, um die Änderung in der Wellenlänge des genannten reflektierten Lichts zu erkennen.

11. Gerät nach einem der vorhergehenden Ansprüche, **dadurch gekennzeichnet, dass** das optische Lesegerät (14, 6) eine Vielzahl von Modulen von optischen Lesegeräten (6a - 6d) umfasst, in mehreren verschiedenen Winkelpositionen um das Rohr (9) herum und auf einem und dmselben horizontalen Plan verteilt, zur Durchführung einer Messung in diesen verschiedenen Winkelpositionen angeordnet.

12. Gerät nach einem der vorhergehenden Ansprüche, **dadurch gekennzeichnet, dass** das optische Lesegerät (14, 6) einen oder mehrere Spiegel (605, 608, 609, 612, 613, 616, 617) umfasst, die derart um das Rohr (9) gelagert sind (9), um das aus der Quelle (601) kommende Licht (603) zu einer Vielzahl von um das Rohr verteilten Messwinkelpositionen (99a - 99d) hin zu reflektieren, und/oder um das Licht zu spiegeln, das durch den Rohrinhalt zu einem und demselben Sensor hin, von einer Vielzahl von Winkelpositionen um das Rohr ausgehend reflektiert wird.

13. Verfahren zur Erkennung des Stands der verschiedenen Phasen (91, 92, 93), die in mindestens einem Rohr (9) oder Behälter vorliegen, das für das automatisierte Bestücken der verschiedenen Schächte mindestens eines Behälters (80) für eine automatisierte Analysevorrichtung (8) bestimmt ist, wobei das genannte Verfahren umfasst:
- eine Verlagerung (D12b), gemäß einer gradlinigen Bewegung und auf bekannte Weise, des genannten Rohrs (9) entlang eines optischen Lesegeräts (16, 4) oder des genannten optischen Lesegeräts entlang des genannten Rohrs;
- Aufzeichnung von Daten, die die vertikale Position mindestens einer Phasenänderung innerhalb des Inhalts des genannten Rohrs darstellen;
wobei die genannte Aufzeichnung eine Aufzeichnung der Lichtmenge aus einer farbigen (601) und durch den Inhalt (99) des genannten Rohrs auf mindestens einen bestimmten Punkt (99a - 99d) reflektierten (604a - 604d) Quelle umfasst, entlang des genannten Rohrs beweglich,
wobei das genannte Verfahren **dadurch gekennzeichnet ist, dass** es durch ein Erkennungsgerät (1) umgesetzt wird, das:
zum einen mit einem derart ausgebildeten Befestigungsteil (111, 112) ausgestattet ist, sodass es selber anstelle eines Behälters mit Mikroplatte-Format gehalten und/oder gehandhabt werden kann; und
zum anderen Einrichtungen (12, 13) zum Verlagern, gemäß einer gradlinigen Bewegung (D12) und auf bekannte Weise, des genannten Rohrs (9) entlang eines optischen Lesegeräts (14, 6) oder des genannten optischen Lesegeräts entlang des genannten Rohrs.

14. Verfahren nach dem vorhergehenden Anspruch, wobei das genannte Verfahren außerdem umfasst:
- einen Schritt zur Positionierung (712) eines Roboterglieds (81), einen Platz im Mikroplatte-Format umfassend, welcher das genannte Erkennungsgerät (1) aufnimmt, sodass eine Erkennung bei einem bestimmten Rohr (9), das aus einer Vielzahl von im Wesentlichen parallel zu einander gelagerten und innerhalb eines Haltegestells (90) angeordneten Rohren (9n) gewählt wird, durchgeführt werden kann;
- einen Erkennungsschritt (713) der Stände des gewählten Rohrs, mit:
- entweder einem Arbeitsschritt zum Herausnehmen nach oben außerhalb des genannten Behälters und mit einer Verlagerung des gewählten Rohrs im Vergleich zum optischen Lesegerät,
- oder einem Arbeitsschritt zur Verlagerung des optischen Lesegeräts durch Einführung nach unten entlang des gewählten Rohrs,
- oder einer Kombination aus diesen Arbeitsschritten.

15. Verfahren nach einem der Ansprüche 13 bis 14, **dadurch gekennzeichnet, dass** es außerdem eine Analyse der reflektierten Lichtmenge (604a - 604d) umfasst, zur Erfassung der Position mindestens eines Endes des Rohrs (9) und/oder zur Ermittlung der Länge des genannten Rohrs eingestellt.

## Claims

1. Apparatus (1) for detecting the level of the different phases (91, 92, 93) present in at least one tube (9) or vessel that is transparent or at least partially transparent, intended for the automated filling of at least one analysis container (80) for an automated analysis system (8), **characterized in that** it comprises:
- means (12, 13) for displacing said tube (9) in a known movement (D12) along an optical reader (14, 6), or said optical reader along said tube;
- data recording means showing the vertical position of at least one phase change within the content of said tube;
said apparatus operating by measuring the variation in wavelength of a light reflected (604a to 604d) off the content (99) of said tube (9), and being equipped with a fixing portion (112) arranged to allow it to be itself held and/or handled instead of a container (8) with the microplate format.

2. Apparatus according to the preceding claim, **characterized in that** it comprises a base (11) the lower periphery or footprint of which has a geometry (111, 112) compatible with the microplate format;
and **in that** the displacement means (12, 13) are arranged to have access to the tube (9) to be read via at least one rectilinear displacement (D12b) of the tube (9) or of the optical reader situated in a region (110) within and below said footprint.

3. Apparatus according to any one of the preceding claims, **characterized in that** the displacement means (12, 13) have at least one so-called retracted position in which they do not extend below or outside the microplate-format footprint (111).

4. Apparatus according to any one of the preceding claims, **characterized in that** the displacement means (12, 13) comprise gripping means (13) of the tube (9) displaced by a mechanism (12) having mobile rods (121, 122, 123, 124), in a single plane of displacement (P12) having a vertical component and around it pivot joints (1213, 1223, 1212, 1221) having axes perpendicular to said plane of displacement, producing a pantograph for displacement of the gripping means (13) or of the optical reader in a rectilinear direction (D12a, D12b) included in said plane of displacement and parallel to the axis of the tube (9) to be read.

5. Apparatus according to any one of the preceding claims, **characterized in that** it comprises gripping means (13) of the tube (9), which have a determined geometry to allow them to be inserted from above (D12a) around a tube arranged within a plurality of tubes (9 to 9n) that are substantially parallel inside a holding rack (90).

6. Apparatus according to any one of the preceding claims, **characterized in that** it comprises within the microplate-format footprint (111) at least one through-opening (114), and **in that** the displacement means (12, 13) are arranged for displacing the gripping means (13) with the tube (9) through said opening (during or before or after) at least partially during the course of a reading displacement (D12b).

7. Apparatus according to any one of the preceding claims, **characterized in that** the displacement means displace the optical reader along a tube situated below said apparatus, said displacement means and said optical reader together having a determined geometry to allow them to be displaced from above along a tube arranged inside a plurality of substantially parallel tubes within a holding container.

8. Apparatus according to any one of the preceding claims, **characterized in that** the optical reader (14, 6) comprises at least one sensor (607, 611, 614, 619) detecting the wavelength of the light (604a to 604d) reflected by the content (99) of the tube (9) in a determined restricted zone (99a to 99d), mobile along said tube during the reading displacement (D12b).

9. Apparatus according to any one of the preceding claims, **characterized in that** it comprises at least one light source arranged to illuminate a restricted zone of the content of the tube, or means of restricting (606, 6010, 614, 618) the illumination and/or the reading to a restricted zone (99a to 99d) of the content (99) of the tube (9).

10. Apparatus according to any one of the preceding claims, **characterized in that**
on the one hand, it comprises one or more light sources (601) emitting only in a determined portion of the light spectrum, and
on the other hand, the optical reader (14, 6) comprises one or more reading modules (6a to 6d) each comprising one single-pixel sensor (607, 611, 615, 619) sensitive to the colour of said light source,
the recording means being arranged in order to use the amount of reflected light (604a to 604d) received by the sensor(s) to recognise the change in the wavelength of said reflected light.

11. Apparatus according to any one of the preceding claims, **characterized in that** the optical reader (14, 6) comprises a plurality of optical reading modules (6a to 6d) distributed in several different angular positions around the tube (9) and in a single horizontal plane, arranged in order to carry out a measurement in these different angular positions.

12. Apparatus according to any one of the preceding claims, **characterized in that** the optical reader (14, 6) comprises one or more mirrors (605, 608, 609, 612, 613, 616, 617) arranged around the tube (9) so as to reflect the light (603) originating from the source (601) to a plurality of angular measurement positions (99a to 99d) distributed around the tube, and/or so as to send the light reflected by the content of the tube to a single sensor from a plurality of angular positions around the tube.

13. Method for detecting the level of the different phases (91, 92, 93) present in at least one tube (9) or vessel intended for filling the different wells of at least one container (80) for an automated analysis system (8), said method comprising:
- a displacement (D12b) of said tube (9) in a rectilinear movement and in a known manner, along an optical reader (16, 4), or of said optical reader along said tube;
- recording of data representing the vertical position of at least one phase change within the content of said tube;
said recording comprising recording the amount of light originating from a source (601) coloured and reflected (604a to 604d) by the content (99) of said tube at at least one determined point (99a to 99d), mobile along said tube,
said method being **characterized in that** it is implemented by a detection apparatus (1) which:
on the one hand, is equipped with a fixing portion (111, 112) arranged to allow it to be itself held and/or handled instead of a microplate-format container, and
on the other hand, comprises means (12, 13) for displacing said tube (9) in a rectilinear movement and in a known manner (D12b) along an optical reader (14, 6), or said optical reader along said tube.

14. Method according to the preceding claim, said method comprising moreover:
- a step of positioning (712) a robot arm member (81) comprising a slot at microplate format for receiving said detection apparatus (1), so as to be able to carry out a detection on a determined tube chosen (9) within a plurality of substantially parallel tubes (9n) arranged within a holding rack (90);
- a step of detecting (713) the levels of the chosen tube, comprising:
- either an operation of upward extraction of said container and a displacement of the chosen tube in relation to the optical reader,
- or an operation of displacement of the optical reader by downward insertion along the chosen tube,
- or a combination of these operations.

15. Method according to any one of claims 13 to 14, **characterized in that** it comprises moreover an analysis of the amount of reflected light (604a to 604d), parametered in order to detect the position of at least one end of the tube (9), and/or in order to calculate the length of said tube.
